# EUROPEAN PATENT APPLICATION

(11) **EP 1 236 713 A1**
(43) Date of publication of application: **04.09.2002**
(21) Application number: 00974890.6
(22) Date of filing: 09.11.2000
(51) Int. Cl.: C07C 303/30, C07C 309/66, C07C 45/41, C07C 47/277, C07C 51/367, C07C 59/64, C07K 5/075

(54) **NOVEL INTERMEDIATE FOR SWEETENER WITH HIGH SWEETNESS AND PROCESS FOR PRODUCING THE SAME**

(30) Priority: 18.11.1999 JP 32810099
(71) Applicant: Ajinomoto Co., Inc., Tokyo 104-8315 (JP)
(72) Inventor: KAWAHARA,Shigeru Amino Science Lab.Ajinomoto Co.In, Kanagawa-ken 210-0801 (JP); MORI,Kenichi Amino Science Lab.Ajinomoto Co., Inc., Kanagawa-ken 210-0801 (JP); NAGASHIMA,Kazutaka Amino Science Lab.AjinomotoCo., Kanagawa-ken 210-0801 (JP); TAKEMOTO,Tadashi Amino Science Lab.Ajinomoto Co.In, Kanagawa-ken 210-0801 (JP)
(74) Representative: Nicholls, Kathryn Margaret
(86) International application number: PCT/JP00/07913
(87) International publication number: WO 01/038297

(57) **Abstract**

From a butyric acid derivative which can be easily and efficiently produced by reacting a hydroxyl-protective derivative of 2-methoxy phenol, wherein the hydroxyl group of 2-methoxy phenol is protected in the form of a sulfonic acid ester (sulfonate), with a 3-methylcrotonic acid in the presence of an acid, a 3-(3-hydroxy-4-methoxyphenyl)-3-methylbutyric acid can be obtained by converting the substituent group at the 3-position of the benzene ring in the butyric acid derivative to a hydroxyl group. From the compound, further by means of a reaction for converting a carboxyl group into a formyl group, a 3-(3-hydroxy-4-methoxyphenyl)-3-methylbutyl aldehyde can be easily produced, and the aldehyde derivative can be easily changed into a compound, which is excellent as a sweetener with a high potency of sweetness, by a reductive alkylation reaction with aspartame.

## Description

### Technical Field

The present invention relates to intermediates for various productions in a food, a pharmaceutical product and the like, particularly to a novel aldehyde derivative and a novel butyric acid derivative useful as a production intermediate for an N-[N-[3-(3-hydroxy-4-methoxyphenyl)-3-methylbutyl]-L-α-aspartyl]-L-phenylalanine 1-methyl ester important as a sweetener having a high potency (degree) of sweetness, processes for the productions thereof, uses thereof as an intermediate for the production thereof, and novel intermediates for the production thereof.

### Background Art

In recent years, as eating habits have been improved to a high level, fatness caused by excessive sugar intake and diseases accompanied by fatness have been at issue. Accordingly, the development of a low-calory sweetener (sweetening agent) that replaces sugar has been strongly in demand. As a sweetener that is widely used at present, there is aspartame which is excellent in safety and quality of sweetness, and however, is somewhat problematic in stability.

Under these background, an N-[N-[3-(3-hydroxy-4-methoxyphenyl)-3-methylbutyl]-L-α-aspartyl]-L-phenylalanine 1-methyl ester represented by the following figure has been found as a sweetener which is excellent in stability and moreover is better by far in potency of sweetness, i.e., has the advantage in cost per a sweet taste, by a part of the present inventors and the like.

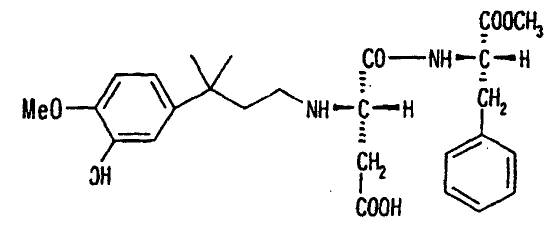

### Problem to be solved by Invention

As a process for production of N-[N-[3-(3-hydroxy-4-methoxyphenyl)-3-methylbutyl]-L-α-aspartyl]-L-phenylalanine 1-methyl ester important as a sweetener described above, a process for
alkylating reductively a β-O-benzyl-α-L-aspartyl-L-phenylalanine methyl ester with a 3-(3-benzyloxy-4-methoxyphenyl)-3-methylbutyl aldehyde followed by removing the benzyl group of a protecting group therefrom was thought by the present inventors. However, according to the process, the aldehyde which is an intermediate for the production, is synthesized in the reaction which needs 7 reaction steps of so many steps from the 3-hydroxy-4-methoxy acetophenone as the starting material, as shown in the following reaction process 1, and therefore it is hardly said that the process is to be an industrially profitable means for reaction.

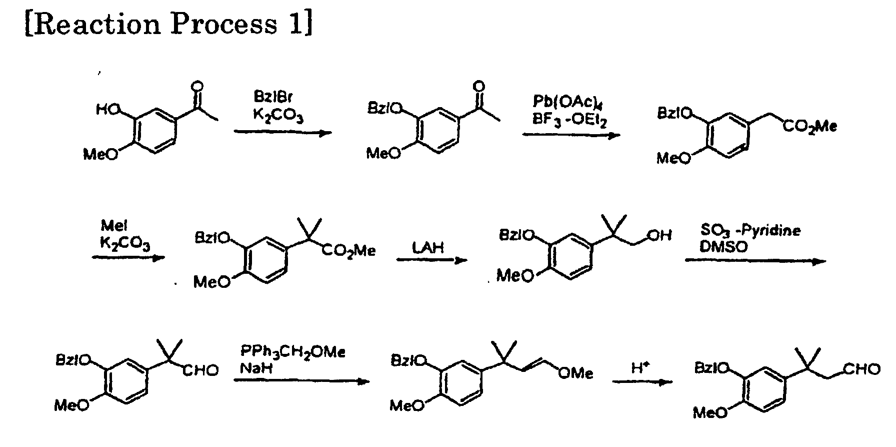

Under these circumstances stated above, a development of a process for producing industrially and easily the aspartyl dipeptide ester derivative described above is requested.

A problem to be solved by the present invention is to provide a process for producing industrially and efficiently the N-[N-[3-(3-hydroxy-4-methoxyphenyl)-3-methylbutyl]-L-α-aspartyl]-L-phenylalanine 1-methyl ester excellent as a sweetener having a high potency of sweetness, and in particular a development of an intermediate for the production therefor, and a conventional or simple process for production thereof.

### Disclosure of Invention

The present inventors have studied much earnestly to solve the above problem, and as a result succeeded in newly synthesizing a 3-(3-hydroxy-4-methoxyphenyl)-3-methylbutyl aldehyde and found that the compound is extremely excellent as an intermediate for the production of sweetener having a high potency of sweetness described above. Further, they have made a study of an efficient process for production of the compound, and as a result found a process shown in the following reaction process 2 as a suitable process therefor. For example, it was found that as shown in this process, as for the compound in question, by subjecting a 3-methylcrotonic acid to a Friedel-Crafts reaction in the presence of an acid with a hydroxyl-protective derivative of 2-methoxy phenol represented by the general formula (1), where the hydroxyl group in the 2-methoxy phenol is protected in the form of a sulfonic acid ester thereof, and thereafter taking apart, for example hydrolyzing or the like an ester moiety of sulfonic acid, the 3-(3-hydroxy-4-methoxyphenyl)-3-methylbutyric acid can be easily produced. It was found that this butyric acid derivative is extremely excellent as an intermediate for the aldehyde derivative described above.

That is to say, it has been found that the 3-(3-hydroxy-4-methoxyphenyl)-3-methylbutyl aldehyde obtained by converting the carboxylic acid corresponding to the compound in question to an aldehyde is extremely excellent as an intermediate for the production of N-[N-[3-(3-hydroxy-4-methoxyphenyl)-3-methylbutyl]-L- α -aspartyl]-L-phenylalanine 1-methyl ester important as a sweetener.

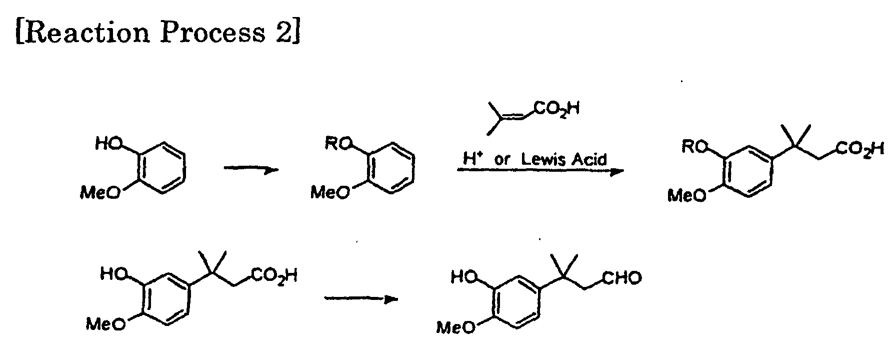

On the basis of the above such various findings, the present invention has reached its completion.

That is to say, the present invention exists in the following contents.
[1]
   A process for producing a 3-substituted-phenyl-3-methylbutyric acid represented by the following general formula (2), which comprises:
   reacting a hydroxyl-protective derivative of 2-methoxy phenol where the hydroxyl group in the 2-methoxy phenol is protected in the form of a sulfonic acid ester thereof, represented
      by the following general formula (1),
      with a 3-methylcrotonic acid in the presence of an acid.

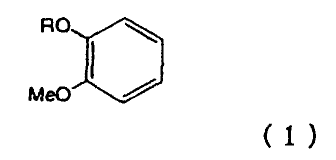

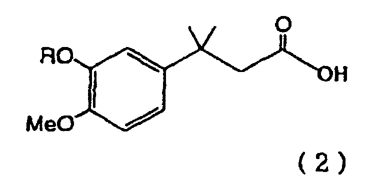

   In the above formulae, R denotes a protecting group of sulfonyl type (-SO₂-R'), and Me denotes a methyl group, respectively.
   In the protecting group of sulfonyl type, for the R', a branched chain or a straight chain (linear) alkyl group having 1 to 10 carbon atoms, which may have substituent group(s); an aryl group having 6 to 15 carbon atoms, which may have substituent group(s); and an aralkyl group having 7 to 20 carbon atoms, which may have substituent group(s) are cited,
   In such case, the above alkyl group includes its derivative fluorinated wherein in the alkyl group, at least a part of the hydrogen atoms bonded to the carbon atom(s) is replaced by fluorine atom(s). For example, a part or whole of the alkyl group may be a fluoroalkyl group.
   And, the above aralkyl group includes its derivative fluorinated wherein in the aralkyl group, at least a part of the hydrogen atoms bonded to the carbon atom(s) except for the carbon atoms located in the aromatic ring is replaced by fluorine atom(s). For example, a part or whole of the alkyl moiety in the aralkyl group may be a fluoroalkyl group.
   In case of the above any group which may have substituent group(s), for the substituent group(s), alkyl group(s) (preferably alkyl group(s) having 1 to 3 carbon atoms), nitro group(s), halogen atom(s) (Cl, Br, F, and the like), trialkyl ammonium group(s) (preferably the alkyl moiety thereof having 1 to 3 carbon atoms) and the like are cited. For specific examples suitable for R, as a protecting group of sulfonyl type, a benzene sulfonyl group (-SO₂-C₆H₅), a p-toluene sulfonyl group (-SO₂-C₆H₄-CH₃), a p-bromobenzene sulfonyl group (-SO₂-C₆H₄-Br), a p-nitrobenzene sulfonyl group (-SO₂-C₆H₄-NO₂), a methane sulfonyl group (-SO₂-CH₃), an ammonioalkane sulfonyl group (-SO₂-(CH₂)ₙ N(CH₃)₃⁺) (n= 0 to 6), a trifluoromethane sulfonyl group (-SO₂-CF₃), a nonafluorobuthane sulfonyl group (-SO₂-C₄F₉), a 2,2,2-trifluoroethane sulfonyl group (-SO₂-CH₂-CF₃) and the like are cited. For the R, in particular, a methane sulfonyl group, a trifluoromethane sulfonyl group, and a p-toluene sulfonyl group are suitable.
[2]
   In the process described above, the process which further comprises:
   subjecting a 3-substituted-phenyl-3-methylbutyric acid represented by the general formula (2) to a reaction for converting the substituent group located at the 3-position of the phenyl group therein to a hydroxyl group to form a 3-(3-hydroxy-4-methoxyphenyl)-3-methylbutyric acid.
[3]
   The above process, wherein the reaction for converting the substituent group located at the 3-position of the phenyl group therein to a hydroxyl group is a hydrolysis reaction for a sulfonic acid ester.
[4]
   The above any processes, wherein R is a methane sulfonyl group (-SO₂-CH₃).
[5]
   In the process [2] described above, the process which further comprises:
   a step of subjecting the compound thus obtained to a reaction for converting a carboxyl group into a formyl group to form an aldehyde.

   At the time when the compound is subjected to a reaction for converting a carboxyl group into a formyl group to form an aldehyde, any one of a process for converting a carboxyl group into a formyl group through half reduction (semi-reduction, partial reduction; a reduction process for converting a carboxyl group into the corresponding formyl group) and a process for converting a carboxyl group into a hydroxymethyl group through reduction, and then converting the hydroxymethyl group into a formyl group through oxidization may be contained therein and performed.
   In these reactions for the conversion, reactions for conversion from a carboxyl group to a formyl group and the like employed in the field of reaction for organic synthesis may be employed.
[6]
   In the process [5] described above, the process which further comprises:
   subjecting the aldehyde thus obtained to a reductive alkylation reaction with an aspartame to form a N-[N-[3-(3-hydroxy-4-methoxyphenyl)-3-methylbutyl]-L- α -aspartyl]-L-phenylalanine 1-methyl ester.
[7]
   A process for producing a N-[N-[3-(3-hydroxy-4-methoxyphenyl)-3-methylbutyl]-L- α -aspartyl]-L-phenylalanine 1-methyl ester, which comprises:
   alkylating reductively an aspartame, by using a 3-(3-hydroxy-4-methoxyphenyl)-3-methylbutyl aldehyde, or a 3-(3-hydroxy-4-methoxyphenyl)-3-methylbutyl aldehyde derived from a 3-(3-hydroxy-4-methoxyphenyl)-3-methylbutyric acid.
[8]
   A benzene derivative represented by the following general formula (3). 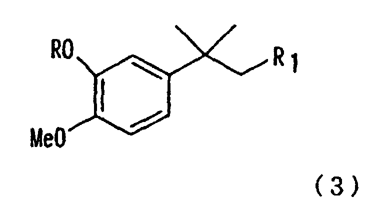
   In the above formula, Me and R denote the same meanings as above described, and R₁ denotes a carboxyl group, a formyl group or a hydroxymethyl group.
[9]
   The above benzene derivative of the following compound:
   3-(3-methanesulfonyloxy-4-methoxyphenyl)-3-methylbutyric acid.

### Embodiments for carrying out Invention

The embodiments for carrying out the present invention are explained in the followings.

As for a process for production of 3-(3-hydroxy-4-methoxyphenyl)-3-methylbutyric acid, based on the above described reaction process 2, the explanations are in the followings.

For protection of the hydroxyl group in a 2-methoxy phenol, the 2-methoxy phenol is converted to a hydroxyl-protective derivative of 2-methoxy phenol represented by the general formula (1) by forming a sufonic acid ester from the 2-methoxy phenol. As for the process therefor, it may be conducted based on the process described in PROTECTIVE GROUPS IN ORGANIC SYNTHESIS (1991, JOHN WILEY & SONS. INC. NEW YORK), p.168-170.

That is, it can be easily achieved by a step of reacting 2-methoxy phenol with the corresponding sulfonic acid anhydride or sulfonic acid chloride in the presence of a base. Such a protecting group for a hydroxyl group can be represented by the formula: -SO₂-R' as the protecting group of sulfonyl type.

For the R', a branched chain or a straight chain (linear) alkyl group having 1 to 10 carbon atoms, which may have substituent group(s); an aryl group having 6 to 15 carbon atoms, which may have substituent group(s); and an aralkyl group having 7 to 20 carbon atoms, which may have substituent group(s) are cited,

In such case, the above alkyl group includes its derivative fluorinated wherein in the alkyl group, at least a part of the hydrogen atoms bonded to the carbon atom(s) is replaced by fluorine atom(s). For example, a part or a whole of the alkyl group may be a fluoroalkyl group.

And, the above aralkyl group includes its derivative fluorinated wherein in the aralkyl group, at least a part of the hydrogen atoms bonded to the carbon atom(s) except for the carbon atoms located in the aromatic ring is replaced by fluorine atom(s). For example, a part or a whole of the alkyl moiety in the aralkyl group may be a fluoroalkyl group.

In case of the above any group which may have substituent group(s), for the substituent group(s), alkyl group(s) (preferably alkyl group(s) having 1 to 3 carbon atoms), nitro group(s), halogen atom(s) (Cl, Br, F, and the like), trialkyl ammonium group(s) (preferably the alkyl moiety thereof having 1 to 3 carbon atoms) and the like are cited. For specific examples suitable for R, as a protecting group of sulfonyl type, a benzene sulfonyl group (-SO₂-C₆H₅), a p-toluene sulfonyl group (-SO₂-C₆H₄-CH₃), a p-bromobenzene sulfonyl group (-SO₂-C₆H₄-Br), a p-nitrobenzene sulfonyl group (-SO₂-C₆H₄-NO₂), a methane sulfonyl group (-SO₂-CH₃), an ammonioalkane sulfonyl group (-SO₂-(CH₂)ₙ N(CH₃)₃⁺) (n= 0 to 6), a trifluoromethane sulfonyl group (-SO₂-CF₃), a nonafluorobuthane sulfonyl group (-SO₂-C₄F₉), a 2,2,2-trifluoroethane sulfonyl group (-SO₂-CH₂-CF₃) and the like are cited. For the R, in particular, a methane sulfonyl group, a trifluoromethane sulfonyl group, and a p-toluene sulfonyl group are suitable.

A reaction of a hydroxyl-protective derivative of 2-methoxy phenol represented by the general formula (1) described above with a 3-methylcrotonic acid is conducted without a solvent, or conducted in an organic solvent with an acid coexistent. As for the organic solvent, if employed, there is no limitations thereto, as far as a material inactive to a ground substance (substrate) for reaction, an acid and a reaction product may be employed therefor. For a preferable solvent, for example, methylene chloride, chloroform, nitrobenzene and the like are cited.

In case that an acid is employed, for the acid to be employed, a proton acid (H⁺), such as sulfuric acid, para (p⁻)toluenesulfonic acid and hydrogen chloride, a Lewis acid (L.A.), such as aluminum chloride and titanium tetrachloride, and the like, and any acids can be employed. Plural acids can be employed, respectively in the proton acid or Lewis acid, if necessary. Further, a proton acid may be possibly employed in combination with a Lewis acid, such as combination of hydrogen chloride with aluminum chloride, if necessary. In addition, an acid fixed firmly onto the surface of the solid phase is desirably employed, because a process for treatment may be simplified. As a preferable acid, an aluminum chloride, a titanium tetrachloride and sulfuric acid, and the like are cited.

Further, an amount of acid to be employed is not limited particularly. In case that much excess of acid is employed to the 3-methylcrotonic acid, the reaction can be conducted in a shorter time to completion. However, from the economical point of view, preferably 5 molar equivalents or less, more preferably 3 molar equivalents or less, and further more preferably 0.1 to 3 molar equivalents or so, of the acid to the 3-methylcrotonic acid may be employed.

As for an amount consumed (employed) of a hydroxyl-protective derivative of 2-methoxy phenol represented by the general formula (1) described above to the 3-methylcrotonic acid, there is no particular limitations thereto. Preferably 0.5 molar equivalents or more, more preferably 1 molar equivalents or more, and further more preferably 1 to 10 molar equivalents or so, of the derivative to the 3-methylcrotonic acid may be employed.

As for a reaction temperature, there is no particular limitations thereto. The higher the reaction temperature is, the more the secondary reaction proceeds. On the other hand, at a low temperature, the reaction speed becomes slow extremely. Therefore, preferably a temperature of 20 to 180 °C or so, and more preferably a temperature of 30 to 100 °C or so may be selected therefor.

In order to obtain a 3-(3-hydroxy-4-methoxyphenyl)-3-methylbutyric acid by decomposing a sulfonic acid ester of 3-substituted-phenyl-3-methylbutyric acid represented by the general formula (2) described above, and obtained in the above process, it can be achieved by a process for reaction of the compound under a basic condition.

As for a kinds of base material employed in such case, there is no particular limitations thereto. A metal hydroxide such as sodium hydroxide, potassium hydroxide and the like is preferable therefor, since it is used industrially for low cost practices.

Further, as for an amount consumed of the base material employed, there is no particular limitations thereto. In order to complete the reaction without delay, preferably 1 molar equivalent or more (1 time mole or more) of the base material thereto may be employed.

In such case, a solvent can be employed, as for the solvent employed in this case, there is no particular limitations thereto. In particular, when a metal hydroxide such as sodium hydroxide, potassium hydroxide and the like is employed, solvent(s) containing at least one solvent selected from alcohol such as methanol, ethanol and isopropyl alcohol, and water is preferable.

Further, as for a reaction temperature at the time when the sulfonic acid ester which is a protective form for the hydroxyl group therein is removed (de-protected) therefrom, there is no particular limitations thereto. In case that at a higher temperature it is conducted, the reaction can be completed in a shorter time. Preferably a temperature of 20 to 150 °C or so, and more preferably 40 to 100 °C or so may be selected from the economical point of view.

The aldehyde can be easily produced by converting the carboxyl group in the carboxylic acid into a formyl group from the 3-(3-hydroxy-4-methoxyphenyl)-3-methylbutyric acid thus obtained.

The 3-(3-hydroxy-4-methoxyphenyl)-3-methylbutyric acid obtained in the above reaction may be directly reduced through half-reduction (semi-reduction, partial reduction; a reduction process for converting a carboxyl group into the corresponding formyl group). Preferably, it is directly reduced through half-reduction on the basis of the process described in Chemistry Letters, issued in 1998, November, p.1143-1144, whereby the starting material can be converted into the 3-(3-hydroxy-4-methoxyphenyl)-3-methylbutyl aldehyde as objective. This process is a process of reducing the 3-(3-hydroxy-4-methoxyphenyl)-3-methylbutyric acid with hydrogen in an organic solvent together with pivalic acid anhydride, palladium acetate, and triphenylphosphine derivative added.

As for the organic solvent employed, there is no particular limitations thereto, as far as a material inactive to a ground substance (starting material) for reaction, a catalyst and a product as objective may be employed therefor. For example, acetone, tetrahydrofuran, toluene, and the like are desirably employed.

As for a quantity consumed (employed) of pivalic acid anhydride, an equimolar (equimole) quantity or more of pivalic acid anhydride to the 3-(3-hydroxy-4-methoxyphenyl)-3-methylbutyric acid may be used. For example, 1 to 5 times molar (moles) quantity or so of pivalic acid anhydride to the 3-(3-hydroxy-4-methoxyphenyl)-3-methylbutyric acid may be used desirably.

As the triphenylphosphine derivative, triphenylphosphine, tritolylphosphine, and the like may be desirably employed. The palladium acetate and the triphenylphosphine derivative are employed as the catalyst, and therefore, for the quantity consumed thereof, only some moles % thereof is sufficient.

As for the reaction temperature, there is no particular limitations thereto. Preferably a temperature range of 40 to 100 °C, and more preferably a temperature range of 60 to 80 °C may employed. At a higher temperature side, the reaction may be promoted, and can be finished (completed) in a shorter time.

Here, as another processes for forming a 3-(3-hydroxy-4-methoxyphenyl)-3-methylbutyl aldehyde from the 3-substituted-phenyl-3-methylbutyric acid represented by the general formula (2) described above, the following two processes can be shown as the exemplification.

### Process 1:

As shown in the following reaction route, the carboxyl group in the 3-substituted-phenyl-3-methylbutyric acid represented by the general formula (2) is reduced completely to a hydroxymethyl group, and then thus obtained hydroxymethyl group is oxidized partially to form a formyl group. In this case, it can be conducted based on a known process for reduction - partial oxidation (Refer to Journal of Organic Chemistry, vol. 48, No. 25, p. 5043-5048, 1983, or the like.).

Next, the formyl group is protected with an acetal group such as dimethyl acetal group, and then the sulfonic acid ester is decomposed for de-protection, and subsequently, the acetal group is removed through acidic hydrolysis for de-protection, whereby the 3-(3-hydroxy-4-methoxyphenyl)-3-methylbutyl aldehyde as objective can be obtained.

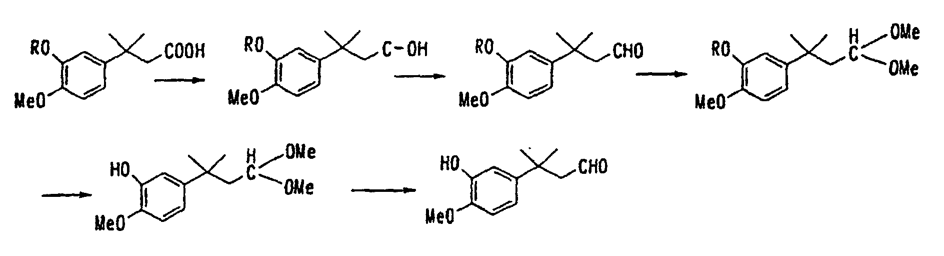

### Process 2:

Further, as shown in the following reaction route, the carboxyl group in the 3-substituted-phenyl-3-methylbutyric acid represented by the general formula (2) is reduced by half-reduction
(semi-reduction, partial reduction; a reduction process for converting a carboxyl group into the corresponding formyl group) to form a formyl group, and thus obtained formyl group is protected with an acetal group such as dimethyl acetal group, and then the sulfonic acid ester is decomposed for de-protection, and subsequently, the acetal group is removed through acidic hydrolysis for de-protection, whereby the 3-(3-hydroxy-4-methoxyphenyl)-3-methylbutyl aldehyde as objective can be obtained.

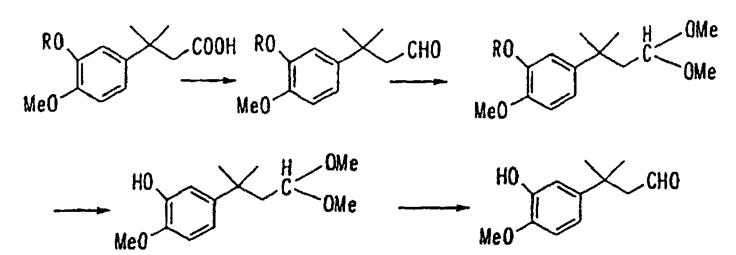

Among these processes, in particular, in case that the 3-(3-hydroxy-4-methoxyphenyl)-3-methylbutyl aldehyde is industrially produced, it is conducted desirably based on the above reaction process 2 from the reasons, such as increase of reaction steps therefor and the like.

On the production of N-[N-[3-(3-hydroxy-4-methoxyphenyl) - 3-methylbutyU-L- α -aspartyl]-L-phenylalanine 1-methyl ester from the 3-(3-hydroxy-4-methoxyphenyl)-3-methylbutyl aldehyde thus obtained, there is no particular difficulty. This aldehyde is alkylated reductively with an α -L-aspartyl-L-phenylalanine methyl ester (aspartame) under a condition for hydrogenation (hydrogen addition), and thereby the aspartyl dipeptide ester derivative can be easily produced.

As for a solvent, if employed, there is no particular limitations thereto, as far as a material inactive to a ground substance (starting material) for reaction, a catalyst and a product as objective may be employed therefor.

A homogeneous organic solvent which can dissolve the aspartame and the above described aldehyde used in the present invention, and which is a single solvent consisted of one kind of organic solvent only or a mixed solvent consisted of plural kinds of organic solvents, or a mixture of such organic solvent(s) with water may be employed therefor.

For the organic solvent, for example, alcohols such as methanol, ethanol and the like, tetrahydrofuran, acetonitrile, dimethylformamide, and the like are cited. From a practical standpoint, alcohol(s) such as methanol or water-containing alcohol(s) such as water-containing methanol is particularly suitable in view of reactivity.

For a catalyst, if employed, a catalyst for hydrogenation (hydrogen addition), such as palladium based catalyst, platinum based catalyst, and rhodium based catalyst, and the like can be preferably cited.

The reductive alkylation reaction used in the present invention can be conducted through hydrogenation (hydrogen addition), and in such case, for the hydrogen pressure, preferably 0.1 to 1.0 MPa or so may be selected.

For a reaction temperature, the condition suitable for a reductive alkylation reaction can be selected. In order to suppress (limit) a secondary reaction and to promote the reaction desired (as objective), a temperature range of 15 to 50 °C or so, and a range for reaction time of 2 to 72 hours or so can be preferably selected.

As for a molar ratio of aspartame to the aldehyde as the starting materials used in the present invention, a range of preferably 0.5 to 1.5 moles or so of the aspartame per 1 mole of the aldehyde can be used for reaction.

Regarding a relation to the known Friedel-Crafts reaction, some explanations are in the following.

As for a Friedel-Crafts reaction using a hydroxyl-protective derivative of 2-methoxy phenol represented by the above general formula (1), it is known already (Refer to Journal of the Agricultural Food Chemistry (1997), vol. 45, No. 6, page 2047-2054.). That is, according to the known process, as shown in the reaction process 3, a phthalic anhydride and a 2-methane sulfonyloxy anisole are subjected to a Friedel-Crafts reaction in the presence of aluminum chloride, and then thus obtained methane sulfonic acid ester is hydrolyzed to be able to obtain a 2-(3-hydroxy-4-methoxybenzoyl)benzoic acid.

According to this process, it is possible to introduce an acyl group to a benzene ring at the para-position to a methoxy group located on the benzene ring, and however the yield therefor is low. Accordingly, it is very much difficult to estimate that the above described Friedel-Crafts reaction with a 3-methylcrotonic acid as in the present invention can proceed at a high yield. In addition, from the above described known process, a content that the butyric acid derivative thus obtained in the present invention is extremely excellent as an intermediate for the production of the above sweetener as the final objective (target) by way of the aldehyde from the standpoints of the yield and also the operation (handling), is not taught, of course and the suggestion therefor also is not given.

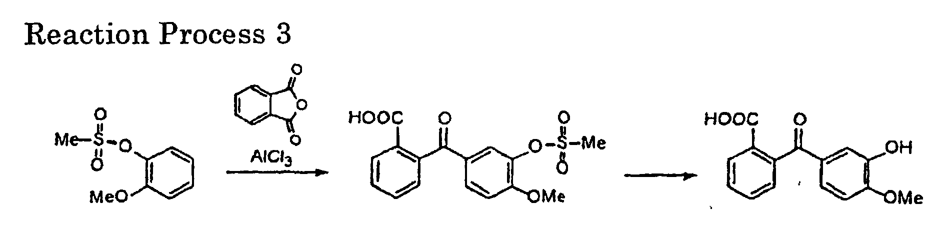

As a process similar thereto, according to the International Patent Publication WO99/1806 Description, it is reported that in the compound having a benzene ring with an electron donating group (electron releasing group) at the ortho-position to a phenolic hydroxyl group located on the benzene ring, the phenolic hydroxyl group is protected in the form of a sulfonic acid ester, and then an electrophilic reagent is reacted with the ester, whereby the reaction proceeds at the para-position to the electron donating group on the benzene ring. However, it is described that in the Friedel-Crafts reaction described in the International Patent Publication, substances such as acid chloride, acid anhydride, 3-chloro-2-methylpropene, and the like, which are all known widely in a general way are reacted in the presence of an acid.

On the other hand, it is difficult to say that a process for using a 3-methylcrotonic acid as an electrophilic reagent under a condition for the Friedel-Crafts reaction is confirmed at full (well-established). For example, in a process for reacting an anisole with a 3-methylcrotonic acid in the presence of aluminum chloride as in the present invention to synthesize a 3-(4-methoxyphenyl)-3-methylbutyric acid, which is described in Journal of Organic Chemistry (1972), vol. 37, No. 36, p. 825-836, the 3-(4-methoxyphenyl)-3-methylbutyric acid obtained gives a yield of 10 % or so to the 3-methylcrotonic acid employed, and therefore this process is not satisfied. It was unthinkable to estimate that with respect to the crotonic acid derivative as found in the present invention, it can produce the objective derivative at an extremely high yield. In the same manner as above, from the above described known process, a content that the butyric acid derivative thus obtained in the present invention is extremely excellent as an intermediate for the production of the above sweetener as the final objective (target) by way of the aldehyde from the standpoints of the yield and also the operation (handling), is not taught, of course and the suggestion therefor also is not given.

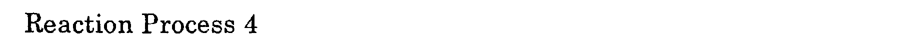

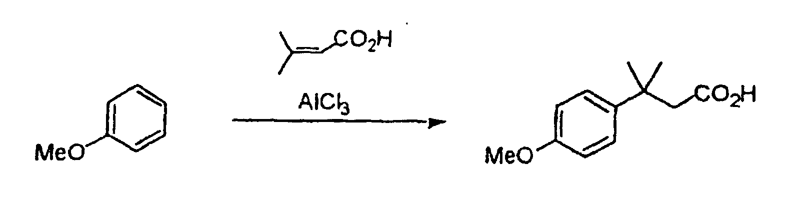

### Examples

The present invention will be explained further in detail with reference to the following Examples.

### (Example 1)

### Synthesis of 3-(3-methanesulfonyloxy-4-methoxyphenyl)-3-methylbutyric acid

To sodium hydroxide in the pellet form (70.8 g), toluene (200 ml) and distilled water (350 ml) were added to dissolve completely the solid material. Subsequently, 2-methoxy phenol (200 g) was added dropwise thereto over a period of 30 minutes, and then methanesulfonyl chloride (184.4 g) was added dropwise thereto at room temperature over a period of 1 hour, and thus obtained mixture as it is, was stirred for 10 hours. Subsequently, the organic layer was separated, and thus obtained solution as it is, was subjected to a step for removal of the solvent by distillation under reduced pressure. Thus obtained residue was subjected a step for distillation under reduced pressure of 1.1 to 1.4 mmHg (146.7 to 186.7 Pa), to obtain unreacted 2-methoxy phenol (64 g) and 2-methanesulfonyloxy anisole (223.6 g, yield: 68 %).

A mixture of 2-methanesulfonyloxy anisole (4.0 g) and 3-methylcrotonic acid (1.0 g) was stirred, and to the thus mixed solution, 95 % sulfuric acid (1.0 g) was added, and the mixture was stirred for 12 hours at 70 °C. Thus obtained reaction solution was cooled to a neighborhood of room temperature, and then distilled water was added thereto to stop the reaction. The reaction solution was extracted with the use of diethyl ether, and the organic layer separated was further extracted with the use of one normal (IN) sodium hydroxide aqueous solution. Subsequently, 6N hydrochloric acid (HCl) solution was further added to the aqueous layer separated, to acidify the solution. Thus obtained aqueous solution was extracted with diethyl ether twice, and thereafter, thus obtained organic layer was concentrated under reduced pressure to obtain a crude 3-(3-methanesulfonyloxy-4-methoxyphenyl)-3-methylbutyric acid (1.1 g; yield in the NMR: 33.8 % to the 3-methylcrotonic acid).
¹H NMR (CDCl₃):
δ:1.44 (s, 6H), 2.60 (s, 2H), 3.15 (s, 3H), 3.87 (s, 3H), 6.94 (d, J=8.5 Hz, 1H), 7.22-7.31 (m, 2H).
ESI-MS:
Calculation: C₁₃H₁₈O₆S=302.34, Analysis: 301.2 (MH⁻).

### (Example 2)

### Synthesis of 3-(3-hydroxy-4-methoxyphenyl)-3-methylbutyric acid

A mixture of 2-methanesulfonyloxy anisole (240 g) and 3-methylcrotonic acid (39 g) was stirred, and to the thus mixed solution, aluminum chloride (104 g) was added, and the mixture was stirred for 5 hours at 70 °C, and further stirred for 2 hours at 100°C. Thus obtained solution was cooled to a neighborhood of room temperature, and then 6N hydrochloric acid (HCl) solution (390 ml) was added thereto, and the mixture was stirred for 3 hours vigorously. The solution obtained was extracted with the use of methylene chloride (300 ml), and the organic layer separated was further extracted with the use of two normal (2N) sodium hydroxide (NaOH) aqueous solution (400 ml). Subsequently, 6N hydrochloric acid (HCl) solution was further added to the aqueous layer separated, to acidify the solution. Thus obtained aqueous solution was extracted with methylene chloride (300 ml) twice, and thereafter, thus obtained organic layer was concentrated under reduced pressure to obtain a residue. Subsequently, to the residue thus obtained, 6N NaOH aqueous solution (300 ml) was added, and the mixture was stirred for 4 hours at 100 °C for mixing. Thus obtained reaction solution was cooled to a room temperature, and then 6N HCl solution was added thereto, to acidify the solution. Thus obtained solution was extracted with ethyl acetate. From the organic solution separated, the solvent therein was removed by distillation under reduced pressure, and thereafter, thus obtained crude crystals were recrystallized with toluene to obtain 3-(3-hydroxy-4-methoxyphenyl)-3-methylbutyric acid as objective (33.2 g; yield: 37.9 % to the 3-methylcrotonic acid).
¹H NMR (CDCl₃):
δ :1.42 (s, 6H), 2.60 (s, 2H), 3.86 (s, 3H), 6.78 (d, J=8.5 Hz, 1H), 6.84 (dd, J=2.2, 8.5 Hz, 1H), 6.95 (d, J=2.2 Hz, 1H).
ESI-MS:
Calculation: C₁₂H₁₆O₄=224.3, Analysis: 223:2 (MH⁻).

### (Example 3)

### Synthesis of 3-(3-hydroxy-4-methoxyphenyl)-3-methylbutyl aldehyde

Into a chemical reactor for hydrogen addition (hydrogenation) under elevated pressure, 3-(3-hydroxy-4-methoxyphenyl)-3-methylbutyric acid (13.6 g), pivalic acid anhydride (22.8 g) and acetone (100 ml) were filled, and thereafter the mixture was bubbled with nitrogen gas for 30 minutes to substitute nitrogen gas completely for the gas in the system of reaction, whereby the system was filled with nitrogen gas. Palladium acetate (137 mg) and tetrahydrofuran solution (5 ml) of tri(p-tolyl) phosphine (930 mg) prepared previously, were added thereto, and then the mixture was stirred under hydrogen pressure of 5 MPa at 80 °C for 24 hours. From thus obtained reaction solution, acetone was removed by vaporization. The remaining residue was subjected to a purification process with the use of a column chromatography to obtain 3-(3-hydroxy-4-methoxyphenyl)-3-methylbutyl aldehyde (10.2 g, yield: 80 %).
¹H NMR (CDCl₃):
δ :1.41 (s, 6H), 2.61 (d, J=3.0 Hz, 2H), 3.87 (s, 3H), 6.72 - 6.84 (m, 2H), 6.98 (d, J=1.9 Hz, 1H), 9.49 (t, J=3.0 Hz, 1H).
ESI-MS:
Calculation: C₁₂H₁₆O₃=208.3, Analysis: 207.2 (MH⁻).

### (Example 4)

### Synthesis of N-[N-[3-(3-hydroxy-4-methoxyphenyl)-3-methylbutyl]-L-α-aspartyl]-L-phenylalanine 1-methyl ester

A 3-(3-hydroxy-4-methoxyphenyl)-3-methylbutyl aldehyde (6.677 g, 25.2 mmol) was dissolved in 80 % methanol aqueous solution (272 ml), and aspartame (8.446 g, 27.8 mmol) was added thereto to prepare a slurry solution. 10 % Palladium on activated carbon in the water content of 50 % (2.86 g) was added thereto under nitrogen stream, and then hydrogen was substituted for the gas inside the system of reaction, and the mixture obtained as it is, was stirred for 24 hours at 25 °C. After the substitution of nitrogen for the gas in the system of reaction, the catalyst was removed by filtration. Water (190 ml) was added to the filtrate, and the solution was extracted with toluene (250 ml) twice. The methanol/water layer separated was concentrated under reduced pressure to about one second (1/2) of the amount thereof (half as much) by weight, and then, the solution was cooled gradually from 75 °C to 5 °C to precipitate crystals. The crystals separated were dissolved in 50 % methanol aqueous solution (260 ml) at 75 °C and cooled to 5 °C to precipitate crystals. The crystals were dried under reduced pressure to obtain N-[N-[3-(3-hydroxy-4-methoxyphenyl)-3-methylbutyl]-L- α -aspartyl]-L-phenylalanine 1-methyl ester (8.46 g, 17.1 mmol, yield: 67.6 % to the aldehyde) as the white crystals. The purity in the HPLC was 98 %.

The physical data on the N-[N-[3-(3-hydroxy-4-methoxyphenyl)-3-methylbutyl]-L- α -aspartyl]-L-phenylalanine 1-methyl ester are in the followings.
¹H NMR (DMSO-d₆):
δ:1.14 (brs, 6H), 1.54 - 1.68 (m, 2H), 2.04 - 2.22 (m, 3H), 2.24 - 2.34 (dd, 1H), 2.84 - 2.94 (dd, 1H), 3.00 - 3.08 (dd, 1H), 3.31 - 3.36 (m, 1H), 3.59 (s, 3H), 3.71 (s, 3H), 4.46 - 4.55 (m, 1H), 6.60 - 6.65 (dd, 1H), 6.73 (s, 1H), 6.80 (d, 1H), 7.10 - 7.28 (m, 5H), 8.45 (d, 1H), 8.75 (brs, 1H).
ESI-MS: 487.3 (MH⁺).

### Effect of Invention

By using a 3-(3-hydroxy-4-methoxyphenyl)-3-methylbutyl aldehyde or a 3-(3-hydroxy-4-methoxyphenyl)-3-methylbutyric acid used as an intermediate for the production in the present invention, and in case of the butyric acid derivative, the butyric acid derivative is converted into the aldehyde corresponding to the butyric acid derivative, and the aldehyde derivative is subjected to a process for reductive alkylation reaction with an aspartame, whereby the N-[N-[3-(3-hydroxy-4-methoxyphenyl)-3-methylbutyll-L- α -aspartyl]-L-phenylalanine 1-methyl ester important as a sweetener having a high potency of sweetness can be easily produced industrially and efficiently.

The aldehyde derivative and butyric acid derivative described above are novel compounds, and these compounds can be easily and efficiently produced through a process which comprises:
reacting a hydroxyl-protective derivative of 2-methoxy phenol where the hydroxyl group in the 2-methoxy phenol is protected in the form of a sulfonic acid ester thereof, with a 3-methylcrotonic acid in the presence of an acid;
removing (eliminating) a moiety of the protecting group therein described above by means of hydrolysis or the like to convert it into a hydroxyl group; and further
converting the carboxylic acid obtained into an aldehyde, where necessary.

Therefore, owing to (thanks to) the process in the present invention, the sweetener with a high potency of sweetness described above can be produced industrially and advantageously.

Some novel compounds useful as intermediates for the production thereof, such as the aldehyde derivative, the butyric acid derivative and the like described above, are provided.

## Claims

1. A process for producing a 3-substituted-phenyl-3-methylbutyric acid represented by the following general formula (2), which comprises:
reacting a hydroxyl-protective derivative of 2-methoxy phenol where the hydroxyl group in the 2-methoxy phenol is protected in the form of a sulfonic acid ester thereof, represented by the following general formula (1),
with a 3-methylcrotonic acid in the presence of an acid. 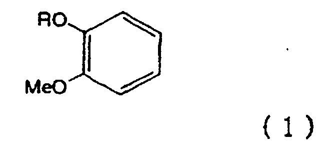 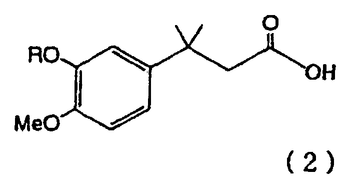
In the above formulae, R denotes a protecting group of sulfonyl type, and Me denotes a methyl group, respectively.

2. The process as defined in claim 1, wherein said protecting group of sulfonyl type is one represented by the general formula:
-SO₂-R'.
In the above formula, R' denotes a straight chain or a branched chain alkyl group having 1 to 10 carbon atoms, which may have substituent group(s); an aryl group having 6 to 15 carbon atoms, which may have substituent group(s); or an aralkyl group having 7 to 20 carbon atoms, which may have substituent group(s),
and the alkyl group includes its derivative fluorinated wherein in the alkyl group, at least a part of the hydrogen atoms bonded to the carbon atom(s) is replaced by fluorine atom(s), and the aralkyl group includes its derivative fluorinated wherein in the aralkyl group, at least a part of the hydrogen atoms bonded to the carbon atom(s) except for the carbon atoms located in the aromatic ring is replaced by fluorine atom(s).

3. The process as defined in claim 1, wherein said protecting is any substituent group selected from a benzenegroup of sulfonyl type sulfonyl group, a p-toluene sulfonyl group, a p-bromobenzene sulfonyl group, a p-nitrobenzene sulfonyl group, a methane sulfonyl group, an ammonioalkane sulfonyl group, a trifluoromethane sulfonyl group, a nonafluorobuthane sulfonyl group, and a 2,2,2-trifluoroethane sulfonyl group.

4. The process as defined in claim 1, wherein said protecting group of sulfonyl type is any substituent group selected from a methane sulfonyl group, a trifluoromethane sulfonyl group, and a p-toluene sulfonyl group.

5. The process as defined in claim 1, wherein said protecting group of sulfonyl type is a methane sulfonyl group.

6. A process for producing a 3-(3-hydroxy-4-methoxyphenyl)-3-methylbutyric acid, which comprises:
obtaining a 3-substituted-phenyl-3-methylbutyric acid represented by the general formula (2) based on the process as defined in any one of claims 1 to 5, and
subjecting the compound thus obtained to a reaction for converting the substituent group at the 3-position of the phenyl group therein to a hydroxyl group.

7. A process for producing a 3-(3-hydroxy-4-methoxyphenyl)-3-methylbutyric acid, which comprises:
obtaining a 3-substituted-phenyl-3-methylbutyric acid represented by the general formula (2) based on the process as defined in any one of claims 1 to 5, and
subjecting the compound thus obtained to a hydrolysis reaction for a sulfonic acid ester to convert the substituent group at the 3-position of the phenyl group therein to a hydroxyl group.

8. A process for producing a 3-(3-hydroxy-4-methoxyphenyl)-3-methylbutyl aldehyde, which comprises:
obtaining a 3-(3-hydroxy-4-methoxyphenyl)-3-methylbutyric acid based on the process as defined in claim 6, and
subjecting the compound thus obtained to a reaction for converting a carboxyl group into a formyl group.

9. A process for producing a N-[N-[3-(3-hydroxy-4-methoxyphenyl)-3-methylbutyl]-L- α -aspartyl]-L-phenylalanine 1-methyl ester, which comprises:
obtaining a 3-(3-hydroxy-4-methoxyphenyl)-3-methylbutyl aldehyde based on the process as defined in claim 8, and
subjecting the compound thus obtained to a reductive alkylation reaction with an aspartame.

10. A process for producing a 3-(3-hydroxy-4-methoxyphenyl)-3-methylbutyl aldehyde, which comprises:
obtaining a 3-(3-hydroxy-4-methoxyphenyl)-3-methylbutyric acid based on the process as defined in claim 7, and
subjecting the compound thus obtained to a reaction for converting a carboxyl group into a formyl group.

11. A process for producing a N-[N-[3-(3-hydroxy-4-methoxyphenyl)-3-methylbutyl]-L- α -aspartyl)-L-phenylalanine 1-methyl ester, which comprises:
obtaining a 3-(3-hydroxy-4-methoxyphenyl)-3-methylbutyl aldehyde based on the process as defined in claim 10, and
subjecting the compound thus obtained to a reductive alkylation reaction with an aspartame.

12. A process for producing a 3-(3-hydroxy-4-methoxyphenyl)-3-methylbutyl aldehyde, which comprises:
obtaining a 3-substituted-phenyl-3-methylbutyric acid represented by the general formula (2) based on the process as defined in claim 1, and then from the compound,
converting the substituent group at the 3-position of the compound thus obtained into a hydroxyl group, and the carboxyl group thereof into a formyl group, respectively.

13. A process for producing a N-[N-[3-(3-hydroxy-4-methoxyphenyl)-3-methylbutyl]-L- α -aspartyl]-L-phenylalanine 1-methyl ester, which comprises:
obtaining a 3-(3-hydroxy-4-methoxyphenyl)-3-methylbutyl aldehyde based on the process as defined in claim 12, and
subjecting the compound thus obtained to a reductive alkylation reaction with an aspartame.

14. A benzene derivative represented by the following general formula (3). 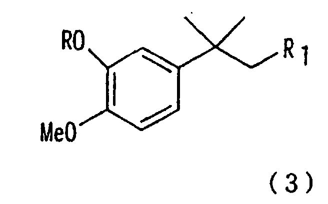 In the above formula, R denotes a protecting group of sulfonyl type, Me denotes a methyl group, and R₁ denotes a carboxyl group, a formyl group or a hydroxymethyl group, respectively.

15. The benzene derivative as defined in claim 14, wherein in said formula,
R denotes a protecting group represented by the general formula: -SO₂-R', and
R' denotes a straight chain or branched chain alkyl group having 1 to 10 carbon atoms, which may have substituent group(s); an aryl group having 6 to 15 carbon atoms, which may have substituent group(s); or an aralkyl group having 7 to 20 carbon atoms, which may have substituent group(s),
and the alkyl group includes its derivative fluorinated wherein in the alkyl group, at least a part of the hydrogen atoms bonded to the carbon atom(s) is replaced by fluorine atom(s), and the aralkyl group includes its derivative fluorinated wherein in the aralkyl group, at least a part of the hydrogen atoms bonded to the carbon atom(s) except for the carbon atoms located in the aromatic ring is replaced by fluorine atom(s).

16. The benzene derivative as defined in claim 14, wherein in said formula,
R₁ denotes a carboxyl group, and
R denotes any substituent group selected from a benzene sulfonyl group, a p-toluene sulfonyl group, a p-bromobenzene sulfonyl group, a p-nitrobenzene sulfonyl group, a methane sulfonyl group, an ammonioalkane sulfonyl group, a trifluoromethane sulfonyl group, a nonafluorobuthane sulfonyl group, and a 2,2,2-trifluoroethane sulfonyl group

17. The benzene derivative as defined in claim 14, wherein in said formula,
R₁ denotes a carboxyl group, and
R denotes any substituent group selected from a methane sulfonyl group, a trifluoromethane sulfonyl group, and a p-toluene sulfonyl group.

18. The benzene derivative as defined in claim 14, wherein in said formula, R₁ denotes a carboxyl group, and R denotes a methane sulfonyl group.

19. A compound of 3-(3-methanesulfonyloxy-4-methoxyphenyl)-3-methylbutyric acid.
